(19) Europäisches Patentamt
European Patent Office
Office européen des brevets

(11) **EP 3 509 728 B1**

(12) **EUROPÄISCHE PATENTSCHRIFT**

(45) Veröffentlichungstag und Bekanntmachung des Hinweises auf die Patenterteilung:
**30.11.2022 Patentblatt 2022/48**

(21) Anmeldenummer: **17798098.4**

(22) Anmeldetag: **30.10.2017**

(51) Internationale Patentklassifikation (IPC):
**B01D 65/00** (2006.01)      **B01D 65/02** (2006.01)
**B01D 69/10** (2006.01)

(52) Gemeinsame Patentklassifikation (CPC):
**B01D 69/10; B01D 65/003; B01D 65/022;**
B01D 46/0028; B01D 2325/28; B01D 2325/38

(86) Internationale Anmeldenummer:
**PCT/EP2017/001267**

(87) Internationale Veröffentlichungsnummer:
**WO 2018/077476 (03.05.2018 Gazette 2018/18)**

(54) **LUFTFILTER, VERFAHREN ZU SEINER HERSTELLUNG UND SEINE VERWENDUNG**

AIR FILTER, METHOD FOR ITS MANUFACTURE AND ITS USE

FILTRE À AIR, MÉTHODE DE SA FABRICATION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.10.2016 DE 102016013019**

(43) Veröffentlichungstag der Anmeldung:
**17.07.2019 Patentblatt 2019/29**

(73) Patentinhaber: **Sartorius Stedim Biotech GmbH 37079 Göttingen (DE)**

(72) Erfinder:
• **LOEWE, Thomas**
**37077 Göttingen (DE)**
• **FRIESE, Thomas**
**99752 Bleicherode (DE)**
• **HANDT, Sebastian**
**37127 Dransfeld (DE)**
• **STRAUSS, Mario**
**37431 Bad Lauterberg (DE)**
• **NIKOLOUDIS, Paschalis**
**37079 Göttingen (DE)**

(74) Vertreter: **Novagraaf International SA Chemin de l'Echo 3 1213 Onex, Geneva (CH)**

(56) Entgegenhaltungen:
**WO-A1-00/04984      WO-A1-97/19743
WO-A1-2012/031653    DE-C1- 4 234 816
DE-C1- 19 729 456    JP-A- 2002 058 974
JP-A- 2006 289 174**

**Beschreibung**

Die vorliegende Erfindung betrifft einen Luftfilter, ein Verfahren zur Herstellung des Luftfilters und die Verwendung des Luftfilters.

Im Stand der Technik offenbart sind Filterelemente, umfassend ein poröses Flächengebilde, welches poröse Membranlagen und poröse Trenn- beziehungsweise Verstärkungslagen umfasst, sowie ein oder mehrere Verankerungselemente, worin das Flächengebilde eingebettet ist. Beispielsweise sind die Flächengebilde röhrenförmig oder sternförmig plissiert. Die beiden Enden der Flächengebilde sind in Endkappen, welche als Verankerungselemente fungieren, eingebettet. Die Einbettung erfolgt üblicherweise durch Eintauchen der Randbereiche des Flächengebildes in die aufgeschmolzene Verankerung. Die porösen Trennlagen sind gemäß dem Stand der Technik Polyester-Spinnvliese, beispielsweise Polyethylenterephthalat-Spinnvliese, nachfolgend als PET-Spinnvliese bezeichnet, welche für den Bau von gammasterilisierbaren plissierten Filterelementen verwendet werden können. Polyester-Spinnvliese sind bis zu einer Strahlendosis von ca. 1000 kGy gammastabil. Die porösen Membranlagen derartiger Filterelemente sollen durch Gammastrahlung sterilisierbar sein.

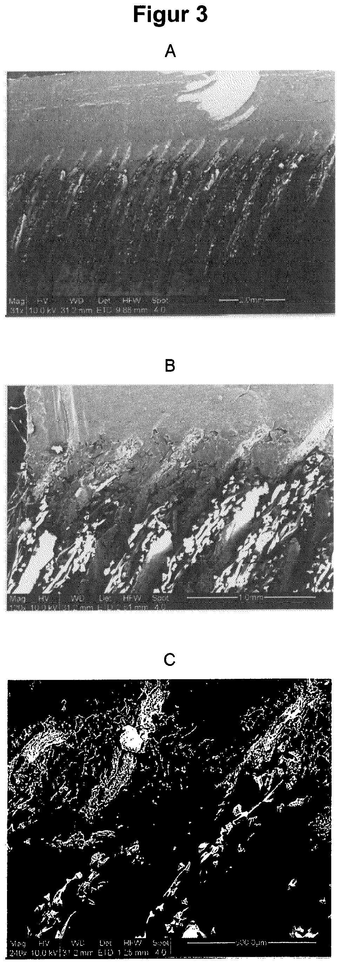

Nachteilig auf die Verarbeitung im Aufbau von Filterelementen zu Steril-Membranfiltern wirkt sich allerdings der hohe Schmelzpunkt der PET-Spinnvliese von etwa 250 °C auf das zu fertigende Endprodukt aus. So kann es unter Umständen (unter anderem aufgrund der Dicke der PET-Einzelfasern) zur Bildung von Undichtigkeiten beziehungsweise Bypässen im Bereich der Einbettung kommen. Beim Eintauchen der Randbereiche des Flächengebildes in die aufgeschmolzene Verankerung führen nicht schmelzende PET-Fasern zu einer unvollständigen Abdichtung (siehe Figur 3). Besonders stark kommt dieser Defekt beim Aufbau einlagiger Membranfilterelemente zum Tragen, da diese im Vergleich zu zweilagigen Membranfilterelementen eine geringere mechanische Stabilität aufweisen und daher beim Einbetten in die geschmolzene Verankerung keinen übermäßigen Kräften ausgesetzt werden können.

Es ist mit den herkömmlichen porösen Trennlagen mit hoher Schmelztemperatur nicht möglich, eine erhöhte mechanische Stabilität durch Erhöhen der Faserdicke und/oder der Dicke der Trennlage zu erreichen. Nachdem die herkömmlichen Trennlagen erst bei höherer Temperatur aufschmelzen, kann bei hohen Dicken der Fasern und/oder der Trennlagen durch unzureichendes Aufschmelzen der Trennlage keine zufriedenstellende Verbindung mit den Verankerungselementen eines Filterelementes erzielt werden. Das Auftreten von Undichtigkeiten/Bypässen würde hierdurch noch verstärkt werden.

Der vorliegenden Erfindung liegt die Aufgabe zugrunde, einen strahlungsresistenten Luftfilter ohne Bypässe sowie ein Herstellungsverfahren für den Luftfilter bereitzustellen.

Diese Aufgabe wird durch die in den Ansprüchen gekennzeichneten Ausführungsformen gelöst.

In einem Aspekt wird ein poröses Flächengebilde mit mindestens einer der folgenden Lage A und mindestens einer der folgenden Lage B beschrieben:

A: eine poröse Membran; und
B: eine poröse Trenn- beziehungsweise Verstärkungslage, welche aus Polyolefin-Kernmantelfasern aufgebaut ist, wobei das Mantelmaterial eine geringere Schmelztemperatur besitzt als das Kernmaterial;
wobei das poröse Flächengebilde eine Strahlungsresistenz von mindestens 20 kGy aufweist.

Im erfindungsgemäßen Luftfilter ist die poröse Trennlage B aus Polyolefin-Kernmantelfasern aufgebaut. Vorzugsweise besteht die poröse Trennlage B aus Polyolefin-Kernmantelfasern. Besonders bevorzugt ist das Mantelmaterial Polyethylen. Polyethylen zeichnet sich durch eine hohe Strahlungsresistenz aus, was positiv zur Strahlungsresistenz des vom erfindungsgemäßen Luftfilter umfassten Flächengebildes beiträgt. Zudem besitzt Polyethylen einen relativ niedrigen Schmelzpunkt. Dadurch kann gewährleistet werden, dass in der Herstellung eines Filterelementes beim Verankern des Flächengebildes in einem Verankerungselement das Mantelmaterial im Bereich des Verankerungselementes vollständig aufschmilzt. Überraschenderweise wurde festgestellt, dass durch Aufschmelzen des Mantelmaterials der Kernmantelfasern die Trennlage B zurückschrumpft, sodass im Bereich der Verankerungselemente die Membranlage A weiter in das zumindest teilweise aufgeschmolzene Material der Verankerungselemente hineinragt als die Trennlage B. Dies führt zu einer festen Einbettung, bei mehreren Membranlagen A sogar zu einer innigen Verzahnung zwischen dem Material des Verankerungselementes und dem Flächengebilde, wodurch Bypässe im Bereich der Verankerungselemente des Filterelementes vermieden werden. Die Vermeidung von Bypässen ist insbesondere bei der Herstellung von Sterilfiltern essentiell. Das Kernmaterial der Kernmantelfasern weist eine höhere Schmelztemperatur als das Mantelmaterial auf. Dadurch ist es möglich, das Mantelmaterial zu schmelzen, ohne die Struktur der porösen Trennlage (abgesehen von dem erwünschten Schrumpfungseffekt) zu verändern.

Darüber hinaus ermöglicht es die spezifische Struktur des Flächengebildes, das Flächengebilde schonend, das heißt bei niedriger Temperatur, zu plissieren, was insbesondere für den Fall einer hydrophob modifizierten Membranlage A, welche wärmeempfindlich sein kann, von Vorteil ist.

Vorzugsweise beträgt die Differenz der Schmelztemperaturen der Mantel- und Kernmaterialien mindestens 5 °C, besonders bevorzugt mindestens 15 °C, insbesondere bevorzugt mindestens 25 °C. Die Differenz ist beispielsweise nicht größer als 100 °C, vorzugsweise 75 °C, insbesondere bevorzugt nicht größer als 50 °C.

Bevorzugt ist das Kernmaterial Polypropylen (PP), beispielsweise mit einem Schmelzpunkt im Bereich von 150 °C ± 10 °C. Das Mantelmaterial ist vorzugsweise Polyethylen (PE), beispielsweise mit einem Schmelzpunkt im Bereich von 135 °C ± 5 °C, 120 °C ± 5 °C oder 105 °C ± 5 °C. Bevorzugt besteht das Mantelmaterial aus mindestens einem von LDPE (Polyethylen niederer Dichte) und HDPE (Polyethylen höherer Dichte) oder einem Gemisch daraus, wobei HDPE besonders bevorzugt ist.

Bevorzugt ist weiterhin, dass das Kernmaterial Polypropylen (PP) mit einem Schmelzpunkt im Bereich von 160 °C ± 10 °C ist und/oder dass das Mantelmaterial Polyethylen (PE) mit einem Schmelzpunkt im Bereich von 112,5 °C ± 10 °C ist. Besonders bevorzugt ist eine Kombination eines Kernmaterials, welches Polypropylen (PP) mit einem Schmelzpunkt im Bereich von 160 °C ± 5 °C, insbesondere bevorzugt 160 °C ± 2 °C ist, und eines Mantelmaterials, welches Polyethylen (PE) mit einem Schmelzpunkt im Bereich von 112,5 °C ± 5 °C, insbesondere bevorzugt 112,5 °C ± 2 °C, am meisten bevorzugt 112,5 °C ± 1 °C, ist.

Erfindungsgemäß kann der Schmelzpunkt mittels eines Kalorimeters mit Differentialabtastung gemessen werden. Hierfür geeignet ist beispielsweise ein Kalorimeter vom Typ "DSC 214 Polyma" der Firma Netsch.

Das Massenverhältnis der Polymere PP:PE (Kernpolymer:Mantelpolymer) in den Kernmantelfasern kann zwischen 2:98 bis 90:10 variieren, wobei ein Verhältnis von 30:70 bis 70:30, insbesondere 40:60 bis 60:40, beispielsweise 50:50 bevorzugt ist.

Gemäß einer bevorzugten Ausführungsform der vorliegenden Erfindung weisen die Kernmantelfasern ein annähernd kreisförmiges Querschnittsprofil auf. Besonders bevorzugt weist auch die Kernfaser ein annähernd kreisförmiges Querschnittsprofil auf.

Unter dem "Durchmesser" wird erfindungsgemäß die größte Längendimension im Querschnittsprofil verstanden.

Der Durchmesser der Kernmantelfasern beträgt erfindungsgemäß vorzugsweise 10 bis 200 μm, besonders bevorzugt 25 bis 100 μm, insbesondere bevorzugt 30 bis 50 μm. Hierbei ist zu beachten, dass bei großem Durchmesser der Kernmantelfasern, beispielsweise 25 bis 200 μm, bevorzugt 30 bis 200 μm, besonders bevorzugt 50 bis 200 μm, insbesondere bevorzugt 75 bis 200 μm, erfindungsgemäß eine poröse Trennlage B mit hoher Porosität und niedrigem Strömungs- beziehungsweise Luftwiderstand bereitgestellt werden kann. Daher kann durch einen großen Durchmesser der Kernmantelfasern der Strömungswiderstand der porösen Trennlage B verringert werden, was ein entsprechendes vom erfindungsgemäßen Luftfilter umfasstes Flächengebilde besonders geeignet für die Luftfiltration macht.

Der Durchmesser der Kernfasern ohne Mantel beträgt vorzugsweise 1 bis 190 μm, besonders bevorzugt 2 bis 100 μm, insbesondere bevorzugt 5 bis 45 μm.

Die Dicke des Mantels beträgt vorzugsweise 1 bis 150 μm, besonders bevorzugt 2 bis 75 μm, insbesondere bevorzugt 5 bis 15 μm. Unter der "Dicke des Mantels" wird die größte Längendimension, gemessen von der Grenzfläche zwischen Kern und Mantel bis zur äußeren Oberfläche der Kernmantelfaser und senkrecht zur Grenzfläche zwischen Kern und Mantel, verstanden.

Vorzugsweise weist die Trennlage B einen Schrumpf nach Autoklavieren bei einer Temperatur von 121 °C für eine Dauer von 30 Minuten von mindestens 2%, vorzugsweise mindestens 4%, insbesondere bevorzugt mindestens 5% auf. Der Schrumpf wird gemäß der folgenden Gleichung berechnet:

$$\text{Schrumpf [\%]} = 100\,\%\cdot(1-(\text{Länge der Trennlage B nach Autoklavieren / Länge der Trennlage B vor Autoklavieren})$$

Darüber hinaus weist die Trennlage B vorzugsweise einen Schrumpf durch Erwärmen auf 130°C (welcher vom Schrumpf durch Autoklavieren verschieden ist) von höchstens 1,5%, vorzugsweise höchstens 1,0%, auf. Des Weiteren ist bevorzugt, dass die Trennlage B einen Schrumpf durch Erwärmen auf 140°C (welcher vom Schrumpf durch Autoklavieren verschieden ist) von höchstens 2,5%, vorzugsweise höchstens 2%, aufweist. Vorstehende Werte können insbesondere dann gering gehalten werden, wenn die Trennlage B aus einem Polyolefinmaterial (beispielsweise Polyethylen-Polypropylen-Kernmantelfasern) aufgebaut ist.

Der Schrumpf durch Erwärmen auf 130°C bzw. der Schrumpf durch Erwärmen auf 140°C können wie folgt bestimmt werden.

Schrumpf durch Erwärmen auf 130°C bzw. der Schrumpf durch Erwärmen auf 140°C

Ein kreisförmiger Stanzling (Durchmesser 142 mm) wird für 10 Sekunden auf eine Heizplatte mit einer Temperatur von 130°C bzw. 140°C gelegt. Die prozentuale Reduktion des Durchmessers, bezogen auf den ursprünglichen Durchmesser des Stanzlings, wird als Schrumpf durch Erwärmen herangezogen.

Gemäß der vorliegenden Erfindung ist das vom Luftfilter umfasste Flächengebilde plissiert. Besonders bevorzugt ist das Flächengebilde plissiert, wobei die Falten unterschiedliche Faltenhöhen haben, wie beispielsweise in DE 10 2010 056 148 B4 beschrieben. Durch die spezifischen Eigenschaften der porösen Trennlage weist das vom erfindungsgemäßen Luftfilter umfasste Flächengebilde eine ausgezeichnete thermoplastische Verformbarkeit auf. Dadurch kann durch Anwenden von Wärme und mechanischer Kraft ein stabiler Plissierverbund erreicht werden, der sich nur schwer senkrecht zur Plissierung verformen lässt (kein "Ziehharmonika-Effekt"). Die hohe Stabilität erleichtert zudem das Verankern des plissierten Flächengebildes (welches auch als "Plissee" bezeichnet werden kann) in einem Verankerungselement im erfindungsgemäßen Verfahren zur Herstellung eines Luftfilters.

Flächengebilde können eine ausschließlich alternierende Lagenfolge (ABABAB usw.) aufweisen, als auch Lagenfolgen, in denen zwei oder mehr gleichartige Lagen aufeinanderfolgen (zum Beispiel AAB oder BBA). Vorzugsweise sind in dem vom erfindungsgemäßen Luftfilter umfassten Flächengebilde benachbarte Lagen direkt benachbart. Das heißt, dass keine weiteren Lagen zwischen benachbarten Lagen eingeschoben sind und benachbarte Lagen aneinander anliegen. Zudem sind die beiden äußeren Lagen des vom Luftfilter umfassten Flächengebildes durch je eine Lage B gebildet. Besonders bevorzugt besteht das vom erfindungsgemäßen Luftfilter umfasste Flächengebilde aus alternierenden Lagen mit zwei Lagen B als äußerste Lagen, das heißt die Lagenfolge ist $B(AB)_n$, wobei n eine ganze Zahl ist.

Gemäß einer bevorzugten Ausführungsform der vorliegenden Erfindung besteht das Flächengebilde aus der Lagenfolge BAB, das heißt in der vorstehenden bevorzugten Lagenfolge $B(AB)_n$ gilt n = 1. Ein derartiges Flächengebilde wird als "einlagig" bezeichnet, wobei sich diese Bezeichnung nach der Anzahl der Membranlagen A richtet. So wird beispielsweise ein Flächengebilde mit zwei Membranlagen A als zweilagig bezeichnet. Das vom erfindungsgemäßen Luftfilter umfasste einlagige Flächengebilde aus der Lagenfolge BAB eignet sich besonders zur Filtration von Lösungen und Gasen. Besonders geeignet ist das Flächengebilde zur Filtration von Lösungen mit Lösungsmitteln, die hydrophobe Membranen benetzen, z. B. unpolare Lösungsmittel. Insbesondere eignet sich das einlagige Flächengebilde zur Sterilfiltration von Luft.

Gemäß einer weiteren bevorzugten Ausführungsform weist das vom erfindungsgemäßen Luftfilter umfasste poröse Flächengebilde zwei aufeinanderfolgende Membranlagen A auf. Besonders bevorzugt handelt es sich bei den beiden Membranlagen um zwei unterschiedliche poröse Membranen $A_1$ und $A_2$, z. B. einen Vor- und einen Hauptfilter. $A_1$ und $A_2$ unterscheiden sich beispielsweise in ihrer Porengröße. Besonders bevorzugt besteht das Flächengebilde aus der Lagenfolge $B(AAB)_n$, wobei n eine ganze Zahl und vorzugsweise 1 ist.

Gemäß der vorliegenden Erfindung können die Lagen A und B lose aufeinanderliegen, ohne fest miteinander verbunden zu sein. Es hat sich jedoch als vorteilhaft erwiesen, die poröse Trennlage B und die Membranlage A durch eine physikalische und/oder chemische Verbindung zwischen direkt benachbarten Lagen zu fixieren. Die physikalische und/oder chemische Verbindung kann beispielsweise durch einen Laminiervorgang mit bekannten Vorrichtungen, als auch durch Druck- und Temperatureinwirkung während der Weiterverarbeitung des vom erfindungsgemäßen Luftfilter umfassten Flächengebildes, zum Beispiel zu einem Plissee, erfolgen. Vorzugsweise ist die Membranlage A auf die Lage B auflaminiert, wie es beispielsweise in der DE-C1 42 34 816 offenbart ist. Das Auflaminieren erfolgt vorzugsweise nur punktuell, wodurch eine ausreichende Verbindung zwischen den Lagen A und B und zugleich ein Flächengebilde mit hoher Durchflussleistung erhalten wird. Ein derartiges Auflaminieren kann beispielsweise durch Plissieren mit einer Plissiertemperatur unterhalb der Schmelztemperatur des porösen Flächengebildes und unter Anwenden eines Plissieranpressdruckes erreicht werden. Insbesondere ist bevorzugt, die Membranlage A nur im Randbereich des Flächengebildes mit der porösen Trennlage B zu laminieren, weil in diesem Fall ein Flächengebilde mit sehr hoher Durchflussleistung erhalten werden kann.

Zum Erzielen einer höchstmöglichen Durchflussleistung ist bevorzugt, dass die Lagen A und B lose aufeinanderliegen, ohne fest miteinander verbunden zu sein. In anderen Worten kann zwischen den Lagen A und B lediglich loser Kontakt bestehen, ohne dass die Lagen fest miteinander verbunden sind. Durch eine physikalische/chemische Verbindung (beispielsweise durch Auflaminieren) können die Oberflächen-Poren der Membran verschlossen werden, wodurch sich die Filtereigenschaften verschlechtern können. Darüber hinaus ist das Erzeugen einer derartigen Verbindung mit einem beträchtlichen Aufwand verbunden. Des Weiteren kann das Weiterverarbeiten (beispielsweise Plissieren/Falten) eines laminierten Flächengebildes zu Defekten in der Membranlage A führen. Es ist zum Beispiel möglich, dass bei einem laminierten Flächengebilde Faserenden der Trennlage B in die Membranlage A eindringen und dadurch Defekte in Form von Einstichlöchern (Pinholes) erzeugt werden. Daher ist ein loses Aufeinanderlegen von A und B insbesondere mit Hinblick auf die Filtrationsleistung vorteilhaft.

Das vom erfindungsgemäßen Luftfilter umfasste Flächengebilde weist eine Strahlungsresistenz von mindestens 20 kGy, vorzugsweise mindestens 50 kGy, besonders bevorzugt mindestens 100 kGy, auf. Die Strahlungsresistenz des vom erfindungsgemäßen Luftfilter umfassten Flächengebildes ist nach oben hin nicht begrenzt und beträgt vorzugsweise höchstens 500 kGy, besonders bevorzugt höchstens 200 kGy. Erfindungsgemäß wird unter einer "Strahlungsresistenz von X kGy" verstanden, dass der Festigkeitsverlust des Flächengebildes nach Gamma-Bestrahlung mit einer Dosis von X kGy höchstens 30 % beträgt (X kann einen beliebigen Wert annehmen und beträgt beispielsweise 20 kGy, 50 kGy, 100 kGy oder 500 kGy). Der Festigkeitsverlust ergibt sich dabei aus der Abnahme des Festigkeitswerts des Flächengebildes nach der Gamma-Bestrahlung mit einer Dosis von X kGy, bezogen auf den Festigkeitswert des Flächengebildes vor der Gamma-Bestrahlung mit dieser Dosis. Beträgt der Festigkeitswert des bestrahlten Flächengebildes 80 % des Festigkeitswertes des unbestrahlten Flächengebildes, beträgt der Festigkeitsverlust erfindungsgemäß 20 %. Die Festigkeitswerte der bestrahlten und unbestrahlten Flächengebilde werden im Sinne der vorliegenden Erfindung durch ihre maximalen Zugkraftwerte $F_{max}$ bei Raumtemperatur beschrieben. Zur Bestimmung von $F_{max}$ wird hierfür eine Probe des Flächengebildes mit den Maßen 20 mm x 150 mm zugeschnitten und horizontal in eine "Zwick Z2.5/TN1S"-Materialprüfmaschine der Fa. Zwick GmbH so eingespannt, dass die freie Probenlänge zwischen den Klemmbacken 4 cm beträgt. Der Kraftaufnehmer "KAP-Z 200N" (Fa. A.S.T., D-01287 Dresden) wird mit einer Geschwindigkeit von beispielsweise 5 cm/min bewegt. Die Messdaten werden von der Gerätesoftware "testXpert" (Fa. Zwick GmbH, D-89079 Ulm) kontinuierlich erfasst und visualisiert. $F_{max}$ wird als Mittelwert von drei bestrahlten Proben des Flächengebildes beziehungsweise drei unbestrahlten Proben bestimmt. Durch die Strahlungsresistenz des vom erfindungsgemäßen Luftfilter umfassten Flächengebildes muss das Flächengebilde beziehungsweise der daraus gebildete erfindungsgemäße Luftfilter zum Zweck der Sterilisierung nicht autoklaviert werden, sondern kann durch energiereiche Strahlung, zum Beispiel Gamma-Strahlung, keimfrei für die Verwendung vorbereitet werden. Demzufolge sind das vom erfindungsgemäßen Luftfilter umfasste Flächengebilde und der erfindungsgemäße Luftfilter durch die Strahlungsresistenz des Flächengebildes in besonderer Weise für die Sterilfiltration von gasförmigen Fluiden und insbesondere zur Sterilfiltration von Luft geeignet.

Gemäß einer bevorzugten Ausführungsform der vorliegenden Erfindung ist die poröse Trennlage B ein Vlies, Gewebe, Gewirke und/oder Gitter, wobei ein Vlies, insbesondere ein Spinnvlies, bevorzugt ist. Durch diese Ausführungsform kann eine besonders hohe Formstabilität der porösen Trennlage B gewährleistet werden.

Gemäß einer bevorzugten Ausführungsform der vorliegenden Erfindung weist die poröse Trennlage B eine Dicke von mindestens 200 μm, besonders bevorzugt von mindestens 300 μm, insbesondere bevorzugt von mindestens 350 μm auf. Durch Dicken in diesem Bereich kann ein vom erfindungsgemäßen Luftfilter umfasstes Flächengebilde mit hoher mechanischer Belastbarkeit und hoher Steifigkeit bereitgestellt werden. Eine hohe Steifigkeit des Flächengebildes ist insbesondere für die Herstellung des erfindungsgemäßen Luftfilters von Vorteil, insbesondere wenn einlagige Flächengebilde mit nur einer Membranlage A verwendet werden. Hierbei ist zu berücksichtigen, dass mit einer größeren Dicke der porösen Trennlage ein größerer Durchmesser der Kernmantelfasern einhergehen sollte.

Die Obergrenze der Dicke unterliegt keiner besonderen Einschränkung, wobei eine Dicke von höchstens 700 μm, vorzugsweise 600 μm nicht überschritten werden sollte.

Gemäß einer bevorzugten Ausführungsform der vorliegenden Erfindung weist die poröse Trennlage B bei einem Druck von $1{,}25 \cdot 10^2$ Pa eine mittlere Luftdurchlässigkeit von 1500 bis 4000 $dm^3/m^2s$, vorzugsweise 2600 bis 3000 $dm^3/m^2s$, besonders bevorzugt 1800 bis 2500 $dm^3/m^2s$, auf. Die mittlere Luftdurchlässigkeit kann wie folgt beschrieben bestimmt werden.

## Bestimmung der mittleren Luftdurchlässigkeit

Zugrunde gelegt werden 10 verschiedene Teststanzlinge eines zu untersuchenden Materials (beispielsweise ein Vlies). Die Messung der Luftdurchlässigkeit jedes der Teststanzlinge mit je einer Fläche von 20 $cm^2$ erfolgt mit einem Luftdurchlässigkeits-Prüfgerät vom Typ "FX 3300 LABOTESTER III" der Firma TEXTEST INSTRUMENTS. Bei jeder der 10 Messungen beträgt der Differenzdruck 125 Pa. Aus der Messung werden 10 Werte erhalten (Einheit: $l/m^2/min$). Die mittlere Luftdurchlässigkeit ist das arithmetische Mittel aus diesen 10 Werten.

Der Fachmann ist auf Grundlage seiner Kenntnisse in der Lage, die Luftdurchlässigkeit der porösen Trennlage B einzustellen.

Die poröse Trennlage B weist vorzugsweise eine mittlere Durchflussporengröße ("mean flow pore size") von mindestens 100 μm, weiter bevorzugt mindestens 110 μm, besonders bevorzugt mindestens 120 μm, insbesondere bevorzugt mindestens 130 μm, noch mehr bevorzugt mindestens 140 μm, auf. Dadurch ist gewährleistet, dass die poröse Trennlage B eine ausreichende Luftdurchlässigkeit aufweist. Die poröse Trennlage B weist vorzugsweise eine mittlere Durchflussporengröße von höchstens 600 μm, weiter bevorzugt höchstens 500 μm, auf. Dadurch ist gewährleistet, dass die Trennlage B eine ausreichende Stabilität aufweist.

Die mittlere Durchflussporengröße kann wie folgt beschrieben bestimmt werden.

## Bestimmung der mittleren Durchflussporengröße ("mean flow pore size")

Zur Bestimmung der mittleren Durchflussporengröße wird zunächst der Luftdurchfluss eines Prüflings im trockenen Zustand bestimmt. Anschließend wird der Prüfling mit einer Flüssigkeit benetzt und über eine Druckrampe mit Druck beaufschlagt. Durch Anstieg des Luftdrucks werden sukzessive (von großen zu kleinen) immer mehr Poren geöffnet. Durch die Korrelation von Druck und Luftdurchfluss können bestimmten Werten des Drucks verschiedene Porengrößen zugeordnet werden. Die mittlere Durchflussporengröße ist die Porengröße (bzw. der dieser zuordenbare Druck), wobei der Luftdurchfluss die Hälfte des Luftdurchflusses des trockenen Prüflings beträgt.

Die Messung wird mit einem Porometer vom Typ "Porolux 500" der Firma IB-FT GmbH durchgeführt. Hierzu wird eine kreisförmige Probe ausgestanzt (18,5 mm Durchmesser), mit Perfluorpolyether benetzt, in das Porometer eingelegt und gemessen (Formfaktor ("shape factor"): 0,715; Anfangsdruck und Enddruck ("inital pressure"; "final pressure"): 0 bzw. 0,02 bar; Anzahl der Schritte ("number of steps"): 20; Zeit/Druckerhöhung: 2000 s/bar). Die Berechnung des ersten Blasenpunktes ("calculation of first bubble point") erfolgte anhand der "first flow"-Methode. Als mittlere Durchflussporengröße wird das arithmetische Mittel aus drei Messungen verwendet.

In einer weiteren bevorzugten Ausführungsform weist die poröse Trennlage ein Flächengewicht von 85 bis 120 g/m$^2$, vorzugsweise 95 bis 110 g/m$^2$, auf.

Die Lage A ist eine hydrophobe poröse Membran. "Hydrophob" bedeutet in diesem Zusammenhang, dass die Oberflächenspannung des Materials, welches die Oberfläche der Membran bildet, einen Kontaktwinkel von mehr als 90° gegenüber Wasser aufweist. Die Messung des Kontaktwinkels kann beispielsweise gemäß ISO15989 erfolgen.

Synthetische Polymere haben in vielen Fällen hydrophobe Oberflächeneigenschaften, die auf die intrinsische Hydrophobizität der synthetischen Materialien zurückzuführen sind. Die Hydrophobizität ist eine Materialkonstante. Sie wird durch die extramolekularen Wechselwirkungen der das Polymer aufbauenden Atomgruppen hervorgerufen.

Aufgrund ihrer gegenüber Wasser niedrigen Oberflächenspannung weisen diese Materialien eine reduzierte Benetzbarkeit mit wässrigen und polaren Medien auf. Für glatte, nicht poröse Oberflächen ist der Kontaktwinkel gegenüber Wasser ein Maß für die Oberflächenspannung. Oberflächen mit einem Kontaktwinkel von mehr als 90° gegenüber Wasser werden als hydrophob bezeichnet. Hydrophobe Substanzen sind nicht mit Wasser mischbar beziehungsweise benetzbar. Die Substanzen sind meist unpolar, und ihre Oberflächenspannung liegt bei 20 °C unter 72 mN/m. Oleophobe Substanzen, die sich durch eine besonders hohe Hydrophobizität auszeichnen, sind nicht mit Ölen und anderen unpolaren Stoffen mischbar oder benetzbar. Ihre Oberflächenspannung ist bei 20 °C kleiner als 21 mN/m. Typische Oberflächenspannungen von Polymeren, die zu Membranen verarbeitet werden, und deren Kontaktwinkel gegenüber Wasser sind in Tabelle 1 aufgeführt.

Tabelle 1: Oberflächenspannungen von glatten, nicht-porösen Polymeren und deren Kontaktwinkel gegenüber Wasser

| Polymer | Oberflächenspannung [mN/m] | Kontaktwinkel gegenüber Wasser [°] |
|---|---|---|
| Polyamid (Nylon) | 75[a] | 49[a] |
| Polyethersulfon (PES) | 58[a] | 54[a] |
| Polyetheretherketon (PEEK) | 49[a] | 71[a] |
| Polyethylen (PE) | 31[b] | 94[b] |
| Polyvinylidenfluorid (PVDF) | 25[b] | 85[b] |
| Polytetrafluorethylen (PTFE) | 18,5[b] | 108[b] |

a) Membrane Science and Technology Series, 11, "Membrane Contactors: Fundamentals, Applications and Potentialities", 2005, E. Drioli et al.

b) J. Appl. Polym. Sci., 1969, 13, 1741-1747, D.K. Owens et al.

Gemäß einer bevorzugten Ausführungsform der vorliegenden Erfindung ist die hydrophobe poröse Membranlage A intrinsisch hydrophob und/oder hydrophob modifiziert, wobei die hydrophobe poröse Membranlage A besonders bevorzugt hydrophob modifiziert ist.

Eine intrinsisch hydrophobe poröse Membranlage A ist erfindungsgemäß aus einem hydrophoben Material aufgebaut. Gemäß einer bevorzugten Ausführungsform der vorliegenden Erfindung ist die poröse Membran aus einem Polymer, ausgewählt aus der Gruppe, bestehend aus Polyethersulfon, Polyvinylidenfluorid und Polyolefinen (z. B. Polyethylen) sowie Gemischen daraus, aufgebaut. Besonders bevorzugt ist die poröse Membran aus Polyethersulfon aufgebaut.

Gemäß der vorliegenden Erfindung kann eine hydrophobe Modifizierung mittels fluorhaltiger Agenzien, beispielsweise in monomerer, oligomerer oder polymerer Form erfolgen. Geeignete Modifizierungsverfahren sind dem Fachmann bekannt. Beispielsweise kann als hydrophob modifizierte Membran die in DE 10 2011 121 018 A1 beschriebene Membran verwendet werden.

Die poröse Membran ist vorzugsweise aus einem Material aufgebaut, dessen Schmelzpunkt mindestens 10 °C, vorzugsweise mindestens 25 °C, besonders bevorzugt mindestens 50 °C höher ist als der Schmelzpunkt des Mantelmaterials. Dadurch kann die strukturelle Integrität der Membranlage A bei der Herstellung des erfindungsgemäßen Luftfilters gewahrt werden.

Gemäß einer bevorzugten Ausführungsform weist die poröse Membran eine mittlere Porengröße von 0,005 bis 10 $\mu$m, vorzugsweise 0,1 bis 1,2 $\mu$m, weiter bevorzugt 0,2 bis 0,65 $\mu$m, auf.

Eine Klassifizierung von porösen Membranen kann anhand ihrer Porengrößen getroffen werden. So wird auf Grundlage der Porengröße generell zwischen Mikrofiltrationsmembranen (mittlere Porengröße: 0,1 bis 10 $\mu$m), Ultrafiltrationsmembranen (mittlere Porengröße: 0,01 bis weniger als 0,1 $\mu$m), Nanofiltrationsmembranen (mittlere Porengröße: 0,001 bis weniger als 0,01 $\mu$m) und Revers-Osmosemembranen (mittlere Porengröße: 0,0001 bis weniger als 0,001 $\mu$m) unterschieden (siehe Shang-Tian Yang, Bioprocessing for Value-Added Products from Renewable Resources, 2007).

Die Bestimmung der mittleren Porengröße erfolgt beispielsweise wie nachfolgend beschrieben.

Verfahren zur Bestimmung der mittleren Porengröße

In Abhängigkeit der zu bestimmenden Porengröße kommen unterschiedliche Verfahren zum Einsatz. Im Bereich der Mikrofiltration wird vornehmlich die Kapillarfluss-Porometrie verwendet. Die Kapillarfluss-Porometrie ist eine Gas/Flüssigkeits-Porosimetrie, bei der die differentiellen Gasdrücke und Flussraten durch eine Membranprobe zuerst im feuchten und anschließend im trockenen Zustand gemessen werden.

Die Membranprobe wird vor der Messung mit benetzender Flüssigkeit so in Kontakt gebracht, dass sämtliche vorhandenen Poren mit dieser Flüssigkeit gefüllt sind. Nach dem Füllen der Poren und dem Einbringen der Probe ist die Messzelle zu verschließen und die Messung zu starten. Der Gasdruck wird nach dem Start der Messung automatisch und schrittweise erhöht und die dem anliegenden Druck entsprechenden Porendurchmesser werden durch den Gasdruck entleert. Dies erfolgt solange, bis der relevante Porenbereich erfasst wurde, das heißt bis auch die kleinsten im Messbereich vorhandenen Poren von Flüssigkeit befreit sind. Danach wird der Druck wieder abgesenkt und die Messung an der nun trockenen Probe automatisch wiederholt. Aus der Differenz beider Druck-Flussrate Kurven wird die Porengrößenverteilung über die Young-Laplace-Gleichung berechnet. (Siehe Shrestha, Aabhash, "Characterization of porous membranes via porometry" (2012), Mechanical Engineering Graduate Theses & Dissertations, Paper 38.)

Die Charakterisierung der Porengrößenverteilung bei Ultra- und Nanofiltrationsmembranen kann nicht durch eine Gas/Flüssigkeits-Porosimetrie vorgenommen werden, da die Poren in diesen Membranen einen deutlich kleineren Durchmesser besitzen. Nach der Laplace-Gleichung steigt der benötigte Druck zur Verdrängung der benetzenden Flüssigkeit bei gegebener Oberflächenspannung auf Größenordnungen bis zu 50 bis 70 bar. Solche Drücke erschweren einerseits die Handhabung und werden teilweise limitiert durch die apparativen Randbedingungen, andererseits ist bei den auf die Membran ausgeübten Drücken und somit resultierenden Kräften eine Veränderung der Struktur zu erwarten und im Extremfall ein Verlust der strukturellen Integrität. Die hier gewonnen physikalischen Eigenschaften bezüglich der Porengrößenverteilung würden nicht denen einer intakten Membran entsprechen. Um eine Charakterisierung dennoch durchführen zu können, wird ein Absenken der Oberflächen- bzw. Grenzflächenspannung der benetzenden und verdrängenden Medien angestrebt. Aus diesem Grund verwendet man eine Flüssig-Flüssig-Verdrängung zweier nicht mischbarer Flüssigkeiten. Die Grenzflächenspannung zwischen diesen beiden Medien ist deutlich geringer als die Oberflächenspannung zwischen einem Gas und einer Flüssigkeit. Zur Verdrängung einer Flüssigkeit durch eine andere gilt analog zur Gas/Flüssigkeits-Porosimetrie die Laplace-Beziehung und es kann ein ähnliches Messverfahren eingesetzt werden, mit dem Unterschied, dass nicht Gasflussraten sondern Flussraten der verdrängenden Flüssigkeit in Abhängigkeit der differentiell Druckerhöhung aufgenommen werden. (Siehe R. Dávila, Characterization of Ultra and Nanofiltration commercial Filters by Liquid-Liquid Displacement Porosimetry, 2013.)

In einem weiteren Aspekt wird ein Verfahren zur Herstellung des porösen Flächengebildes beschrieben, umfassend die Schritte

(1) Bereitstellen der mindestens einen porösen Membran A;
(2) Bereitstellen der mindestens einen poröse Trennlage B;
(3) Anordnen der mindestens einen porösen Membran A und der mindestens einen poröse Trennlage B zu dem porösen Flächengebilde.

Die vorstehenden Ausführungen in Bezug auf das Flächengebilde gelten entsprechend für das Verfahren zu seiner Herstellung.

Gemäß einer bevorzugten Ausführungsform schließt sich nach Schritt (3) ein Schritt (4) des Laminierens der Lage A auf die Lage B an. Geeignete Verfahren zum Laminieren sind dem Fachmann bekannt und beispielsweise in DE-C1 42 34 816 offenbart, wie bereits vorstehend erwähnt. An Schritt (4) kann sich gegebenenfalls der nachfolgend beschriebene Schritt (5) anschließen oder gleichzeitig mit diesem durchgeführt werden.

An Schritt (3) oder Schritt (4) kann sich ein Schritt (5) des Plissierens des Flächengebildes anschließen. Geeignete Plissierverfahren sind dem Fachmann bekannt. Vorzugsweise wird bei einer Temperatur von 70 bis 110 °C plissiert. In diesem niedrigen Temperaturbereich wird das Flächengebilde geschont, was insbesondere für hydrophob modifizierte Membranlagen A von Vorteil ist. Wie bereits vorstehend erwähnt, kann Schritt (4) des Laminierens gleichzeitig mit beziehungsweise durch den Schritt (5) des Plissierens erfolgen.

In einem weiteren Aspekt wird ein Luftfilter beschrieben, umfassend ein poröses Flächengebilde und mindestens ein Verankerungselement, wobei die poröse Membran A des Flächengebildes in ihren Randbereichen fluiddicht in das Verankerungselement eingebettet ist. Eine fluiddichte Einbettung kann insbesondere dann gewährleistet werden, wenn das Flächengebilde in seinen Randbereichen in das Verankerungselement eingebettet ist. Durch die fluiddichte Einbettung werden Bypässe im Bereich der Verankerungselemente vermieden, wie bereits vorstehend erläutert. Dadurch können erfindungsgemäße Luftfilter mit ausgezeichneten Filtereigenschaften bereitgestellt werden. Aufgrund der fluiddichten Verankerung eignet sich der Luftfilter zur Sterilfiltration von Luft.

Weiterhin ist bevorzugt, dass der erfindungsgemäße Luftfilter einen BCT-Wert gemäß ASTM F838-15 von 0 CFU aufweist. "CFU" ist das englischsprachige Akronym für "koloniebildende Einheiten" ("colony forming units").

Gemäß einer bevorzugten Ausführungsform sind Luftfilter mit einer Mindestfilterfläche von 0,15 m$^2$ WIT-testbar ("WIT" steht für "Wasserintrusionstest"). Der WIT-Wert ist abhängig von der Filterfläche und der Lagenfolge. "WIT-testbar" bedeutet, dass beispielsweise ein Luftfilter mit einem einlagigen Flächengebilde der Lagenfolge BAB, wobei A eine hydrophob modifizierte Polyethersulfonmembran ist, und einer Filterfläche von 0,17 m$^2$ einen WIT-Wert von höchstens 5 ml/10 min, vorzugsweise höchstens 4 ml/10 min, insbesondere bevorzugt höchstens 3 ml/10 min, beispielsweise 0,3 ml/10 min oder weniger aufweist. Bei einer Filterfläche von 0,34 m$^2$ und ansonsten gleichen Bedingungen bedeutet "WIT-testbar", dass der WIT-Wert höchstens 6,5 ml/10 min, vorzugsweise höchstens 5,5 ml/10 min, besonders bevorzugt höchstens 4,5 ml/10 min und insbesondere bevorzugt höchstens 0,45 ml/10 min beträgt; bei einer Filterfläche von 0,53 m$^2$ und ansonsten gleichen Bedingungen höchstens 8 ml/10 min, vorzugsweise höchstens 7 ml/10 min, besonders bevorzugt höchstens 6 ml/10 min und insbesondere bevorzugt höchstens 2,6 ml/10 min. Das Verfahren zur Bestimmung des WIT-Wertes ist in den Beispielen angegeben.

Die Form des Flächengebildes im erfindungsgemäßen Luftfilter ist sternförmig plissiert und der Luftfilter röhrenförmig, wie beispielsweise für Filtercapsulen, Filterkerzen oder Rohrmodule der Fall ist.

Vorzugsweise umfasst das Verankerungselement im Bereich der Einbettung ein thermoplastisches Polymermaterial, besonders bevorzugt besteht das Verankerungselement aus einem thermoplastischen Polymermaterial. Dadurch ist es möglich, durch Aufschmelzen des thermoplastischen Polymermaterials im Bereich der Einbettung und anschließendes Aufsetzen des Verankerungselementes auf das Flächengebilde eine besonders stabile und fluiddichte Einbettung/Verankerung zu erzielen.

Der Luftfilter der vorliegenden Erfindung weist zwei Verankerungselemente auf, wobei die Verankerungselemente Endkappen des röhrenförmigen Luftfilters sind. Hierbei ist das vom Luftfilter umfasste Flächengebilde sternförmig plissiert.

Gemäß einer weiteren bevorzugten Ausführungsform weist der Luftfiltert eine Rückstausicherung und einen Kern auf.

Das vom erfindungsgemäßen Luftfilter umfasste Flächengebilde weist eine hydrophobe Membranlage A auf und bevorzugt eine aus ebenfalls hydrophoben Polyolefin-Kernmantelfasern aufgebaute Trennlage B. So ist er zur Filtration von Luft besonders geeignet.

In einem weiteren Aspekt wird ein Verfahren zur Herstellung eines Luftfilters beschrieben, umfassend die Schritte:

(I) Bereitstellen eines porösen Flächengebildes;
(II) Bereitstellen mindestens eines Verankerungselementes;
(III) fluiddichtes Einbetten der Randbereiche der porösen Membran A in das mindestens eine Verankerungselement.

Geeignete Verfahren zum fluiddichten Einbetten sind dem Fachmann bekannt. Beispielsweise kann das Verankerungselement, sofern es im Bereich der Einbettung ein thermoplastisches Polymermaterial umfasst, im Bereich der Einbettung durch Erwärmen aufgeschmolzen werden, gefolgt von einem Schritt des Aufsetzens des Verankerungselementes auf den Randbereich des Flächengebildes. Demgemäß umfasst gemäß einer bevorzugten Ausführungsform der vorliegenden Erfindung der Schritt (III) die Schritte

(IIIa) Aufschmelzen des thermoplastischen Polymermaterials, welches das Verankerungselement im Bereich der Einbettung aufweist; und
(IIIb) Aufsetzen des Verankerungselementes auf den Randbereich des Flächengebildes.

Schritt (IIIb) erfolgt vorzugsweise bei einer Temperatur des aufgeschmolzenen thermoplastischen Polymermaterials von 180 bis 600 °C, besonders bevorzugt 200 bis 400 °C, insbesondere bevorzugt 220 bis 300 °C. Durch Temperaturen in diesem niedrigen Bereich wird eine übermäßige Wärmeeinwirkung auf das Flächengebilde vermieden. Dadurch werden unerwünschte Veränderungen insbesondere der Membranlage A vermieden. Vor allem bei einer hydrophob modifizierten Membranlage A kann durch eine zu hohe Temperatur des thermoplastischen Materials die hydrophobe Modifizierung Schaden nehmen, wodurch die Leistungsfähigkeit des Luftfilters durch teilweise Hydrophilisierung eingeschränkt werden kann.

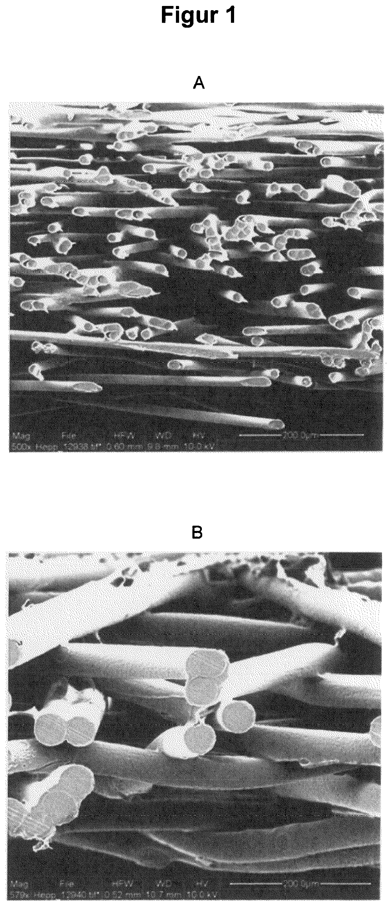

Figur 1 zeigt REM-Aufnahmen ("REM" steht für Rasterelektronenmikroskopie) je einer porösen Trennlage B beispielhafter Flächengebilde gemäß der vorliegenden Erfindung.

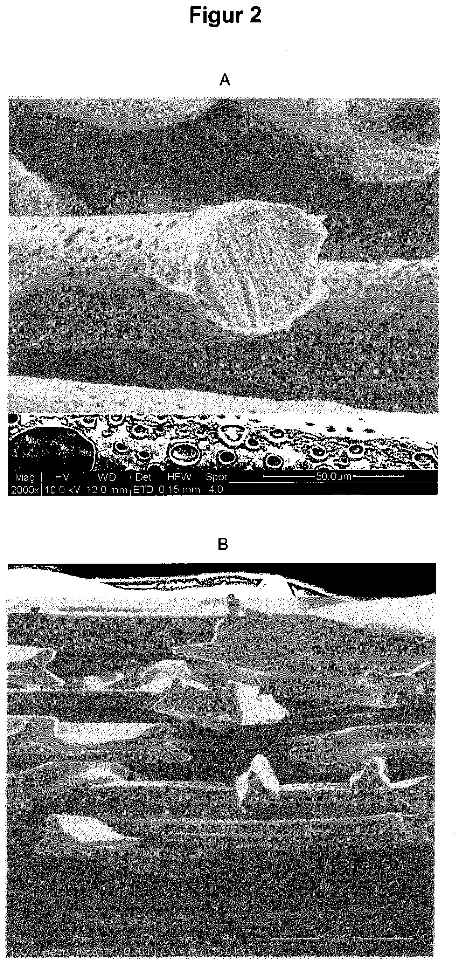

Figur 2 zeigt REM-Aufnahmen eines PP/PE-Kernmantelspinnvlieses bei 2000-facher Vergrößerung (A) und eines PET-Spinnvlieses bei 1000-facher Vergrößerung (B), wobei die Vliese aus Figur 2A und 2B die gleiche Drainagewirkung aufweisen.

Figur 3 zeigt in unterschiedlichen Vergrößerungen REM-Aufnahmen eines Kappenschnitts (Schnitt durch die Endkappen des Filterelementes) eines Filterelementes gemäß dem Stand der Technik, worin anstelle einer porösen Trennlage aus Polyolefin-Kernmantelfasern ein Polyestervlies verwendet wurde. Es ist vor allem in den Vergrößerungen B und C deutlich zu sehen, dass die Polyesterfasern im Einbettungsbereich nicht vollständig aufgeschmolzen sind und das Polyestervlies kaum zurückgeschrumpft ist, wodurch die Bildung von Bypässen ermöglicht wird.

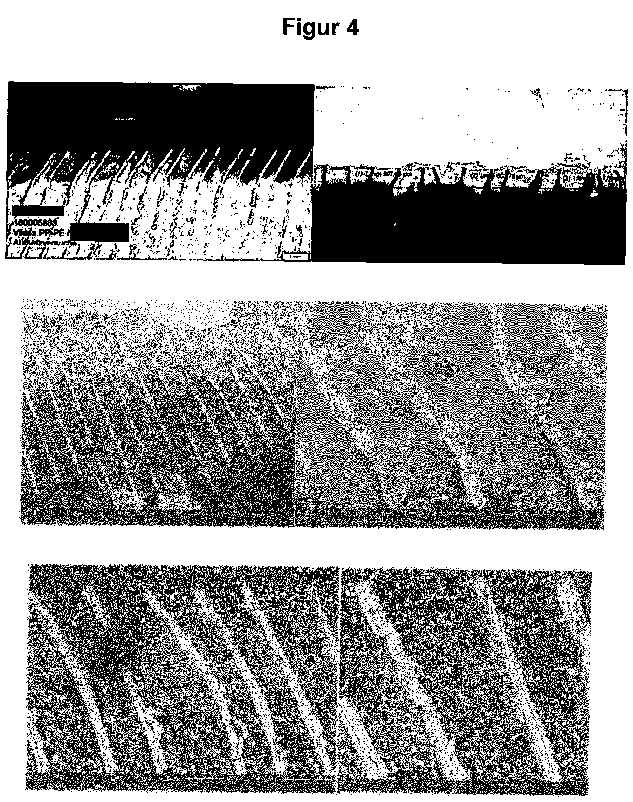

Figur 4 zeigt unterschiedliche Aufnahmen eines Kappenschnitts eines Luftfilters gemäß der vorliegenden Erfindung. Es ist deutlich zu sehen, dass die porösen Trennlagen B zurückgeschrumpft sind und die Membranlagen A im Bereich der Einbettung vollständig umhüllt und mit dem Verankerungselement innig verzahnt sind, wodurch die Bildung von Bypässen vermieden wird

Figur 5 zeigt schematisch den Versuchsaufbau von Beispiel 1 (Wasserintrusionstest), wobei A ein Messgerät (Sartocheck 4 von Sartorius Stedim Biotech GmbH) und C einen Aufbau mit plissierter Filterkerze mit zwei Endkappen und Filtergehäuse B bezeichnen.

Die vorliegende Erfindung wird anhand der folgenden, nicht einschränkenden Beispiele näher erläutert.

**Beispiele**

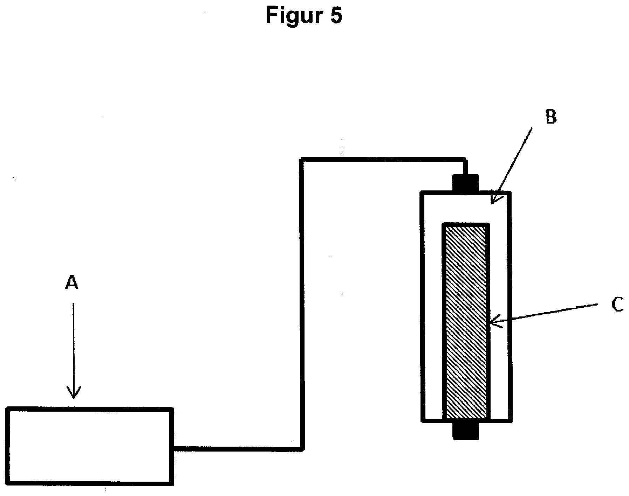

Verschiedene Filtereinheiten gemäß der vorliegenden Erfindung und gemäß dem Stand der Technik (plissierte Filterkerze mit zwei Endkappen und Filtergehäuse B, konkret eine Filtercapsule, deren Aufbau in Figur 5 gezeigt ist) wurden gemäß der folgenden Versuchsbeschreibungen untersucht. Als Ergebnisse wurden der WIT-Wert als Maß für die Integrität der Filtereinheit gegen Wasser, sowie der BCT-Wert (BCT = "bacterial challenge test") als Maß für die Eignung der Filtereinheit für die Sterilfiltration, erhalten.

*Bedingungen Wasserintrusionstest (WIT)*

Die Anströmseite (Upstreamseite) des Filtergehäuses wird mit Wasser gefüllt. Anschließend werden 1,5 bar Druck auf das System gegeben und für 10 min stabilisiert. Während der Testzeit von 10 min erfolgt die Intrusion des Wassers in die erste Schicht der Membran. Der Druckabfall wird gemessen. Aus dem gemessenen Volumen des Wassers, welches benötigt wird, um den Druckabfall zu kompensieren, und dem Druckabfall sowie der bekannten Testzeit wird der WIT-Wert als Maß für die Wasserintrusion berechnet. Der Versuch wird mithilfe eines Messgerätes Sartocheck 4 von Sartorius Stedim Biotech GmbH durchgeführt.

*Bedingungen Biologischer Kontaminationstest (BCT)*

Der BCT wird gemäß ASTM F838-15 durchgeführt. Der verwendete Keim ist *Brevundimonas Diminuta.* Als "steril" wird ein Ergebnis von CFU = 0 gewertet.

In den folgenden Beispielen und Vergleichsbeispielen wurden die folgenden porösen Trennlagen B verwendet.

Tabelle 2

|  | mittlere Vliesdicke [$\mu$m] | Grammatur [g/m$^2$] |
|---|---|---|
| PET 1 | 250 | 45 |
| PET 2 | 250 | 35 |
| PET 3 | 150 | 25 |

(fortgesetzt)

|  | mittlere Vliesdicke [$\mu$m] | Grammatur [g/m$^2$] |
|---|---|---|
| PET 4 | 230 | 30 |
| PET 5 | 110 | 30 |
| PP/PE | 450 | 90 |

Als hydrophob modifizierte Membran wurde eine Membran, wie in DE 10 2011 121 018 A1 beschrieben, verwendet.

Die Ergebnisse der durchgeführten Messungen sind in den folgenden Tabellen 3 bis 7 angegeben. Sofern die Filtereinheiten vor der jeweiligen Untersuchung bestrahlt wurden, ist dies zusammen mit der Strahlendosis angegeben.

Beispiel 1

Die untersuchte Filterkerze, verbaut als Capsule, wies die Lagenfolge BAB auf (PP/PE Kernmantelvlies // hydrophob modifizierte Membran // PP/PE Kernmantelvlies). Untersucht wurden einzelne Individuen (mit Nummern (Nr.) bezeichnet) jeweils baugleicher Chargen.

Die in den nachstehenden Tabellen angegebenen Filtercapsulen der Bauhöhen 7, 9 und 10 wiesen jeweils eine Filterfläche von etwa 0,06 m$^2$, 0,17 m$^2$ und 0,55 m$^2$ auf.

Tabelle 3

| Lagenfolge | Bauhöhe | Charge | Nr. | Keimzahl [CFU] | Behandlung |
|---|---|---|---|---|---|
| BAB | 9 | 121 | 2 | 0 | Bestrahlt 50 kGy |
|  |  | 121 | 28 | 0 |  |
|  |  | 120 | 1 | 0 |  |
|  |  | 120 | 2 | 0 |  |
|  |  | 119 | 14 | 0 |  |
|  |  | 119 | 18 | 0 |  |
| BAB | 10 | 141 | 99 | 0 |  |
|  |  | 141 | 108 | 0 |  |

Alle getesteten Filtercapsulen der Chargen 119, 120, 121 und 141 waren steril (CFU = 0).

Tabelle 4

| Lagenfolge | Bauhöhe | Charge | Nr. | WIT-Wert [ml/10 min] | Behandlung |
|---|---|---|---|---|---|
| BAB | 9 | 121 | 1 | 0,8 | Bestrahlt 50 kGy |
|  |  | 121 | 21 | 0,8 |  |
|  |  | 121 | 38 | 0,8 |  |
|  |  | 120 | 6 | 1,2 |  |
|  |  | 120 | 25 | 1,4 |  |
|  |  | 120 | 38 | 0,9 |  |
| BAB | 10 | 141 | 107 | 2,4 |  |
|  |  | 141 | 115 | 2,3 |  |
|  |  | 141 | 93 | 2,5 |  |

Die Kombination zweier PP/PE Kernmantelvliese mit einer hydrophob modifizierten Membran in der Lagenfolge BAB ist nach Bestrahlung WIT-testbar als auch BCT sterilfiltrierend gemäß ASTM F838-15.

Vergleichsbeispiel 1

Die untersuchten Filterkerzen, verbaut in Capsulen, wiesen die Lagenfolgen PET1 // hydrophob modifizierte Membran // PET1 beziehungsweise PET1 // PET2 // hydrophob modifizierte Membran // PET2 // PET1 auf.

Tabelle 5

| Lagenfolge | Bauhöhe | Charge | Nr. | Keimzahl [CFU] | Behandlun |
|---|---|---|---|---|---|
| PET1//PET2// hydrophob mod. Membran// PET2//PET1 | 9 | 98 | 54 | Belag | Bestrahlt 50 kGy |
| | | | 65 | 362 | |
| | | 24 | 37 | Rasen | |
| | | 23 | 12 | Rasen | |
| | | | 21 | Rasen | |
| | | 177 | 8 | Belag | |
| | | | 16 | Belag | |
| PET1// hydrophob mod. Membran// PET1 | 7 | 458 | 12 | Belag | |
| | 9 | 468 | 4 | Rasen | |

Die Angaben "Belag" beziehungsweise "Rasen" geben einen Keimbefall an, der bereits mit bloßem Auge zu erkennen ist.

Tabelle 6

| Kombination | Bauhöhe | Charge | Nr. | WIT [ml/10min] | Behandlung |
|---|---|---|---|---|---|
| PET1//PET2//hydrophob mod. Membran//PET1 | 9 | 177 | 9 | 15,7 | Bestrahlt 50 kGy |
| | | | 12 | 19,9 | |
| | | | 14 | 27,2 | |
| | | | 18 | 15,8 | |
| PET1//PET2// hydrophob mod. Membran//PET2//PET1 | 9 | 411 | 25 | 31,2 | |
| PET1//hydrophob mod. Membran//PET1 | 9 | 456 | 20 | 20,9 | |
| | | 115 | 9 | 82,1 | |
| | | 113 | 1 | 78 | |
| | | | 5 | 94 | |
| | | 466 | 1 | 14,5 | |
| | | | 2 | 20,2 | |
| | | | 15 | 104,3 | |

Die untersuchten Kombinationen von Polyestervlies mit hydrophob modifizierter Membran sind nach dem Bestrahlen weder WIT-testbar (sehr hohe WIT Ergebnisse) noch BCT-sterilfiltrierend.

Beispiel 2

In diesem Beispiel wurden weitere Filterkerzen gemäß der vorliegenden Erfindung, verbaut als Capsule, und Vergleichs-Filterkerzen mit diversen PET Kombinationen als poröse Trennlage B getestet. Die Ergebnisse sind in der folgenden Tabelle 7 zusammengefasst.

Tabelle 7

| Lagenfolge | Bauhöhe | Charge | Anteil der als steril getesteten Filterkerzen in %; in Klammern: Anzahl der getesteten Kerzen | Behandlung |
|---|---|---|---|---|
| PET1//PET2// hydrophob mod. Membran//PET2//PET1 | 9 | 200 | 70% (10) | unbehandelt |
| PET1//PET4// hydrophob mod. Membran//PET4//PET1 | | 207 | 30% (10) | |
| PET1//PET5// hydrophob mod. Membran//PET5//PET1 | | 208 | 50% (10) | |
| PET1//hydrophob mod. Membran//PET1 | | 239 | 0% (2) | |
| PET1//PET3// hydrophob mod. Membran//PET3//PET1 | | 245 | 30% (10) | |
| PET1//PET1// hydrophob mod. Membran//PET1//PET1 | | 254 | 20% (5) | |
| PP/PE//hydrophob. mod. Membran//PP/PE | 9 | 119 | 100% (10) | Bestrahlt 50 kGy |
| | | 120 | 100% (10) | |
| | | 121 | 100% (10) | |

Wie aus Tabelle 7 ersichtlich, sind die Vergleichsfilterkerzen selbst ohne Gammabestrahlung nicht BCT-sterilfiltrierend. Im Gegensatz dazu bleiben die erfindungsgemäßen Filtereinheiten selbst nach Gammabestrahlung BCT-sterilfiltrierend.

Die vorliegende Erfindung stellt einen Luftfilter bereit, der ein poröses Flächengebilde umfasst, womit verbesserte Filtereinheiten hergestellt werden können. Da das Mantelmaterial des Polyolefin-Kernmantelvlieses bei einer niedrigen Temperatur schmilzt, kann das vom erfindungsgemäßen Luftfilter umfasste Flächengebilde bei niedrigen Temperaturen plissiert und in ein Verankerungsmaterial eingebettet werden. Sofern als poröse Membranlage A eine hydrophob modifizierte Membran verwendet wird, wird die hydrophobe Modifizierung vor Zerfall durch hohe Verarbeitungstemperaturen bewahrt. (Ein Zerfall der Hydrophobisierung kann zur Bildung von hydrophilen Bereichen führen, wodurch sich die Eigenschaften des Filters stark verändern können, was zur Folge haben könnte, dass die Funktion des Luftfilters beeinträchtigt wird. Insbesondere die Luftdurchflussleistung als auch das WIT-Prüfverhalten könnten sich verschlechtern.) Die erfindungsgemäßen Luftfilter sind selbst nach Gammabestrahlung sowohl WIT-testbar als auch BCT-sterilfiltrierend, was für vergleichbare Filterelemente gemäß dem Stand der Technik nicht der Fall ist. Zudem können aus den vom erfindungsgemäßen Luftfilter umfassten Flächengebilden auf schonende Weise besonders stabile Plissierungen erzeugt werden.


**Patentansprüche**

1. Luftfilter, umfassend
   ein poröses Flächengebilde mit mindestens einer der folgenden Lage A und mindestens einer der folgenden Lage B:

   A: eine poröse Membran, wobei die poröse Membran A hydrophob ist; und
   B: eine poröse Trennlage, welche aus Polyolefin-Kernmantelfasern aufgebaut ist, wobei das Mantelmaterial eine geringere Schmelztemperatur besitzt als das Kernmaterial;
   wobei das poröse Flächengebilde eine Gammastrahlungsresistenz von mindestens 20 kGy aufweist, die beiden äußeren Lagen des porösen Flächengebildes durch je eine Lage B gebildet sind, und das poröse Flächengebilde sternförmig plissiert ist,
und
zwei Verankerungselemente, wobei die Verankerungselemente Endkappen des Luftfilters sind und der Luftfilter röhrenförmig ist,
wobei die poröse Membran A in ihren Randbereichen fluiddicht in die Verankerungselemente eingebettet ist.

2. Luftfilter nach Anspruch 1,
wobei das Kernmaterial der porösen Trennlage B Polypropylen und das Mantelmaterial der porösen Trennlage B Polyethylen ist.

3. Luftfilter nach einem der Ansprüche 1 oder 2,
wobei die poröse Membran aus einem Polymer, ausgewählt aus der Gruppe, bestehend aus Polyethersulfon, Polyvinylidenfluorid und Polyolefinen sowie Gemischen daraus, aufgebaut ist.

4. Luftfilter nach einem der Ansprüche 1 bis 3,
welches aus der Lagenfolge BAB oder BAAB besteht.

5. Verfahren zur Herstellung eines Luftfilters nach einem der Ansprüche 1 bis 4, umfassend die Schritte:

(I) Bereitstellen eines porösen Flächengebildes mit mindestens einer der folgenden Lage A und mindestens einer der folgenden Lage B:

A: eine poröse Membran, wobei die poröse Membran A hydrophob ist; und
B: eine poröse Trennlage, welche aus Polyolefin-Kernmantelfasern aufgebaut ist, wobei das Mantelmaterial eine geringere Schmelztemperatur besitzt als das Kernmaterial;

wobei das poröse Flächengebilde eine Gammastrahlungsresistenz von mindestens 20 kGy aufweist, die beiden äußeren Lagen des porösen Flächengebildes durch je eine Lage B gebildet sind, und das poröse Flächengebilde sternförmig plissiert ist;
(II) Bereitstellen der zwei Verankerungselemente, wobei die Verankerungselemente Endkappen des Luftfilters sind und der Luftfilter röhrenförmig ist;
(III) fluiddichtes Einbetten der Randbereiche der porösen Membran A in die Verankerungselemente.

6. Verwendung des Luftfilters nach einem der Ansprüche 1 bis 4 zur Sterilfiltration von Luft.

**Claims**

1. An air filter, comprising
a porous sheet product having at least one of the following ply A and at least one of the following ply B:

A: a porous membrane, wherein the porous membrane A is hydrophobic; and
B: a porous separation ply constructed from polyolefin core-sheath fibers, wherein the sheath material has a lower melting temperature than the core material;
wherein the porous sheet product has a gamma radiation resistance of at least 20 kGy, the two outer plies of the porous sheet product are formed by one ply B each, and the porous sheet product is radially pleated, and
two anchoring elements, wherein the anchoring elements are end caps of the air filter and the air filter is tubular, wherein, in its edge regions the porous membrane A is embedded into the anchoring elements in a fluid-tight manner.

2. The air filter as claimed in claim 1,
wherein the core material of the porous separation ply B is polypropylene and the sheath material of the porous separation ply B is polyethylene.

3. The air filter as claimed in claim 1 or 2,
wherein the porous membrane is constructed from a polymer selected from the group consisting of polyether sulfone, polyvinylidene fluoride and polyolefins and also mixtures thereof.

**4.** The air filter as claimed in any of claims 1 to 3, which consists of the ply sequence BAB or BAAB.

**5.** A method for producing an air filter as claimed in any of claims 1 to 4, comprising the steps of:

(I) providing a porous sheet product having at least one of the following ply A and at least one of the following ply B:

A: a porous membrane, wherein the porous membrane A is hydrophobic; and
B: a porous separation ply constructed from polyolefin core-sheath fibers, wherein the sheath material has a lower melting temperature than the core material;

wherein the porous sheet product has a gamma radiation resistance of at least 20 kGy, the two outer plies of the porous sheet product are formed by one ply B each, and the porous sheet product is radially pleated;
(II) providing the two anchoring elements, wherein the anchoring elements are end caps of the air filter and the air filter is tubular;
(III) embedding the edge regions of the porous membrane A into the anchoring elements in a fluid-tight manner.

**6.** The use of the air filter as claimed in any of claims 1 to 4 for the sterile-filtration of air.

**Revendications**

**1.** Filtre à air, qui comprend
une formation en feuilles poreuse avec au moins une des couches A suivantes et au moins une des couches B suivantes :

A : une membrane poreuse, dans lequel la membrane A poreuse est hydrophobe ; et
B : une couche de séparation poreuse, laquelle est montée en fibres de gaine centrale de polyoléfine, dans lequel le matériau de gaine possède une température de fusion inférieure à celle du matériau central ;
dans lequel la formation en feuilles poreuse présente une résistance au rayonnement gamma d'au moins 20 kGy, les deux couches extérieures de la formation en feuilles poreuse sont chacune formées par une couche B, et la formation en feuilles poreuse est plissée en forme d'étoile,
et
deux éléments d'ancrage, dans lequel les éléments d'ancrage sont des embouts d'extrémité du filtre à air et le filtre à air est tubulaire,
dans lequel la membrane A poreuse, dans ses régions de bord, est intégrée aux éléments d'ancrage de façon étanche aux fluides.

**2.** Filtre à air selon la revendication 1,
dans lequel le matériau central de la couche de séparation B poreuse est du polypropylène et le matériau de gaine de la couche de séparation B poreuse est du polyéthylène.

**3.** Filtre à air selon l'une des revendications 1 ou 2, dans lequel la membrane poreuse est montée dans un polymère, sélectionné dans le groupe constitué du polyéthersulfone, du fluorure de polyvinylidène et des polyoléfines ainsi que de mélanges de ceux-ci.

**4.** Filtre à air selon l'une des revendications 1 à 3, lequel est composé de la séquence de couches BAB ou BAAB.

**5.** Procédé de fabrication d'un filtre à air selon l'une des revendications 1 à 4, qui comprend les étapes consistant à :

I) fournir une formation en feuilles poreuse avec au moins une des couches A suivantes et au moins une des couches B suivantes :

A : une membrane poreuse, dans lequel la membrane A poreuse est hydrophobe ; et
B : une couche de séparation poreuse, laquelle est montée en fibres de gaine centrale de polyoléfine, dans lequel le matériau de gaine possède une température de fusion inférieure à celle du matériau central ;

dans lequel la formation en feuilles poreuse présente une résistance au rayonnement gamma d'au moins 20 kGy, les deux couches extérieures de la formation en feuilles poreuse sont chacune formées par une couche B, et la formation en feuilles poreuse est plissée en forme d'étoile ;

II) fournir les deux éléments d'ancrage, dans lequel les éléments d'ancrage sont des embouts d'extrémité du filtre à air et le filtre à air est tubulaire ;

III) intégrer les régions de bord de la membrane A poreuse aux éléments d'ancrage de façon étanche aux fluides.

6. Utilisation du filtre à air selon l'une des revendications 1 à 4 pour une filtration stérile d'air.

# Figur 1

A

B

# Figur 2

A

B

# Figur 3

A

B

C

# Figur 4

**Figur 5**

# EP 3 509 728 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010056148 B4
- DE 4234816 C1
- DE 102011121018 A1

**In der Beschreibung aufgeführte Nicht-Patentliteratur**

- **E. DRIOLI.** Membrane Contactors: Fundamentals, Applications and Potentialities. *Membrane Science and Technology Series,* 2005, vol. 11
- **D.K. OWENS.** *J. Appl. Polym. Sci.,* 1969, vol. 13, 1741-1747
- **SHANG-TIAN YANG.** *Bioprocessing for Value-Added Products from Renewable Resources,* 2007
- **SIEHE SHRESTHA, AABHASH.** Characterization of porous membranes via porometry. *Mechanical Engineering Graduate Theses & Dissertations,* 2012, 38
- **SIEHE R. DÁVILA.** Characterization of Ultra and Nanofiltration commercial Filters. *Liquid-Liquid Displacement Porosimetry,* 2013